# EUROPEAN PATENT SPECIFICATION

(11) **EP 2 157 971 B1**
(45) Date of publication and mention of the grant of the patent: **11.01.2012**
(21) Application number: 08749545.3
(22) Date of filing: 11.04.2008
(51) Int. Cl.: A61K 31/167, A61K 31/4706, A61K 31/517, A61P 35/00, A61P 35/04, A61P 11/06, G01N 33/574

(54) **EGFR INHIBITORS FOR TREATMENT AND DIAGNOSIS OF METASTATIC PROSTATE CANCER**
EGFR-INHIBITOREN ZUR BEHANDLUNG UND DIAGNOSE VON METASTASIERENDEM PROSTATAKREBS
TRAITEMENT ET DIAGNOSTIC DU CANCER MÉTASTATIQUE DE LA PROSTATE PAR DES INHIBITEURS DE RÉCEPTEUR DE FACTEUR DE CROISSANCE ÉPIDERMIQUE (EGFR)

(30) Priority: 13.04.2007 DK 200700547
(43) Date of publication of application: 03.03.2010
(73) Proprietor: Rikshospitalet- Radiumhospitalet HF, 0027 Oslo (NO); PamGene B.V., 5211 's Hertogenbosch (NL)
(72) Inventor: REE, Anne Hansen, N-1344 Haslum (NO); BRATLAND, Ase, N-0310 Oslo (NO); BOENDER, Pieter Jacob, NL-6522 KJ Nijmegen (NL); RUIJTENBEEK, Robby, NL-3524 BK Utrecht (NL)
(74) Representative: De Clercq, Ann G. Y.
(86) International application number: PCT/EP2008/054432
(87) International publication number: WO 2008/125633

(56) References cited:
- WO-A1-2006/052249
- ANGELUCCI ADRIANO ET AL: "Suppression of EGF-R signaling reduces the incidence of prostate cancer metastasis in nude mice" ENDOCRINE-RELATED CANCER, vol. 13, no. 1, March 2006 (2006-03), pages 197-210, XP008089426 ISSN: 1351-0088
- MIMEAULT MURIELLE ET AL: "Combined targeting of epidermal growth factor receptor and hedgehog signaling by gefitinib and cyclopamine cooperatively improves the cytotoxic effects of docetaxel on metastatic prostate cancer cells" MOLECULAR CANCER THERAPEUTICS, vol. 6, no. 3, March 2007 (2007-03), pages 967-978, XP008089359 ISSN: 1535-7163
- MIMEAULT MURIELLE ET AL: "Novel combination therapy against metastatic and androgen-independent prostate cancer by using gefitinib, tamoxifen and etoposide" INTERNATIONAL JOURNAL OF CANCER, vol. 120, no. 1, January 2007 (2007-01), pages 160-169, XP008089360 ISSN: 0020-7136
- SHAFFER ET AL.: "A phase I/II trial to assess tolerability and efficacy of RAD-001 with gefitinib in patients with glioblastoma multiforme and castrate metastatic prostate cancer" EUROPEAN JOURNAL OF CANCER. SUPPLEMENT, PERGAMON, OXFORD, GB, vol. 3, no. 2, October 2005 (2005-10), page 419, XP005133559 ISSN: 1359-6349
- SEE ET AL: "Bicalutamide 150 mg in Addition to Standard Care Delays Progression to Bone Metastases in Patients with Locally Advanced Prostate Cancer: Analyses From the Second Analysis of the Early Prostate Cancer Program" INTERNATIONAL JOURNAL OF RADIATION: ONCOLOGY BIOLOGY PHYSICS, PERGAMON PRESS, US, vol. 63, 1 October 2005 (2005-10-01), pages S286-S287, XP005083495 ISSN: 0360-3016
- SALZBERG MARC ET AL: "An open-label, noncomparative phase II trial to evaluate the efficacy and safety of docetaxel in combination with gefitinib in patients with hormone-refractory metastatic prostate cancer" ONKOLOGIE, vol. 30, no. 7, 2007, pages 355-360, XP008089357 ISSN: 0378-584X
- TOS A P DEI: "The biology of epidermal growth factor receptor and its value as a prognostic/predictive factor" INTERNATIONAL JOURNAL OF BIOLOGICAL MARKERS, vol. 22, no. 1, Suppl. 4, January 2007 (2007-01), pages S3-S9, XP002520065 ISSN: 0393-6155
- SOHA SALAMA EL SHEIKH ET AL: "PHOSPHORYLATION OF BOTH EGFR AND ERBB2 IS A RELIABLE PREDICTOR OF PROSTATE CANCER CELL PROLIFERATION IN REPONSE TO EGF" NEOPLASIA, DOYMA, BARCELONA, ES, vol. 6, no. 6, 1 November 2004 (2004-11-01), pages 846-853, XP008050753 ISSN: 0212-9787
- NORMANNO N ET AL: "Epidermal growth factor receptor tyrosine kinase inhibitors and bone metastases: Different mechanisms of action for a novel therapeutic application?" ENDOCRINE-RELATED CANCER 200603 GB, vol. 13, no. 1, March 2006 (2006-03), pages 3-6, XP002520066 ISSN: 1351-0088
- ANGELUCCI ADRIANO ET AL: "Osteopontin enhances the cell proliferation induced by the epidermal growth factor in human prostate cancer cells" PROSTATE, vol. 59, no. 2, 1 May 2004 (2004-05-01), pages 157-166, XP002520067 ISSN: 0270-4137
- ODA KANAE ET AL: "A comprehensive pathway map of epidermal growth factor receptor signaling ( ARTICLE NO.: 2005.0010)" MOLECULAR SYSTEMS BIOLOGY, [Online] vol. 1, no. 1, May 2005 (2005-05), XP002520068 ISSN: 1744-4292 Retrieved from the Internet: URL:http://www.nature.com/msb/journal/v1/n 1/pdf/msb4100014.pdf>

## Description

### Field of invention

The present invention relates to Gefitinib for use in treating or preventing metastatic prostate cancer.

### Background

Metastatic prostate cancer is a leading cause of cancer morbidity and mortality. The skeleton is the principal organ for metastasis formation in prostate cancer, and bone metastases are often both painful and debilitating. Androgens are critical regulators of prostate carcinoma growth and progression, but most patients respond only temporarily to androgen ablation therapy, also at bone metastasis sites.

Skeletal metastases from prostate cancer are essentially osteoblastic, as apparent both radiographically and histopathologically, and as consistent with elevated serum level of bone-specific alkaline phosphatase, a marker of osteoblast proliferation, in patients with metastatic prostate cancer [Logothetis & Lin, 2005]. These observations implicate that the biological interaction between the prostate carcinoma cells and osteoblasts contributes to the metastatic progression of prostate cancer.

Insight into regulatory mechanisms underlying establishment of prostate carcinoma cells within an osteoblastic microenvironment might lead to more effective therapies for metastatic disease.

A cascade of events is required for prostate carcinoma cells to metastasize to bone. The questions of how features of prostate carcinoma cells influence osteoblasts and *vice versa,* and how these features facilitate formation of the typical skeletal lesions, are still elusive. Insight into the mechanisms underlying these processes will not only help to explain the predilection of prostate cancer - as opposed to other tumor entities - to an osteoblastic bone microenvironment, but could also lead to development of prediction biomarkers and/or preventive agents or more effective therapies for advanced prostate cancer.

Conceptually, the overall metastatic potential may depend on a set of cellular characteristics that also determine the carcinoma cells' affinity for the bone marrow microenvironment. The elucidation of regulatory mechanisms underlying colonization of bone, however, depends on a careful choice of experimental model and analytical technology.

From a clinical point of view, prostate cancer metastasis to bone is a lengthy and complex disease process, which makes it difficult to find adequate laboratory models to recreate all steps involved [Singh & Figg, 2005]. However, given that regulatory mechanisms implicated in the metastatic phenotype are evoked when the carcinoma cells settle within an osteoblastic microenvironment, an experimental setup addressing how osteoblastic cells may influence prostate carcinoma cell biology upon formation of bone metastasis was invented. As highlighted in a recent review [Singh & Figg, 2005], most of the experimental systems examining this phenomenon are based on rodent models. Importantly, the model systems used by us exclusively utilize cell types of human origin.

### Summary of the invention

To study the regulatory basis underlying establishment of prostate carcinoma cells within an osteoblastic microenvironment, an experimental model system and novel analytical technology were combined to obtain information about functional signaling pathways and networks involved.

We used the human, androgen-sensitive LNCaP prostate carcinoma cell line [Horoszewicz *et al.,* 1983] in coculture with the human, osteoblast-derived OHS cell line [Fodstad *et al.,* 1986], as previously described [Bratland *et al.,* 2003], to simulate the direct cellular interaction.

Factors secreted by osteoblasts have been proposed to stimulate prostate carcinoma cells [Logothetis & Un, 2005]. Monocultured LNCaP cells were therefore treated with OHS-conditioned medium to experimentally replicate the biological context of paracrine influence.

Androgens are critical regulators of prostate carcinoma progression; however, until recently, the regulatory program mediated by the androgen receptor in prostate cancer has been elusive [Dehm & Tindall, 2006]. To simulate the complex processes involved in aberrant activation of the androgen signaling axis in prostate cancer [Scher & Sawyers, 2005; Attard et al., 2006], our experimental setup included LNCaP cells treated with a synthetic androgen analog (R1881) to observe whether androgen receptor-mediated signaling pathways might differ from pathways activated by OHS-directed influence.

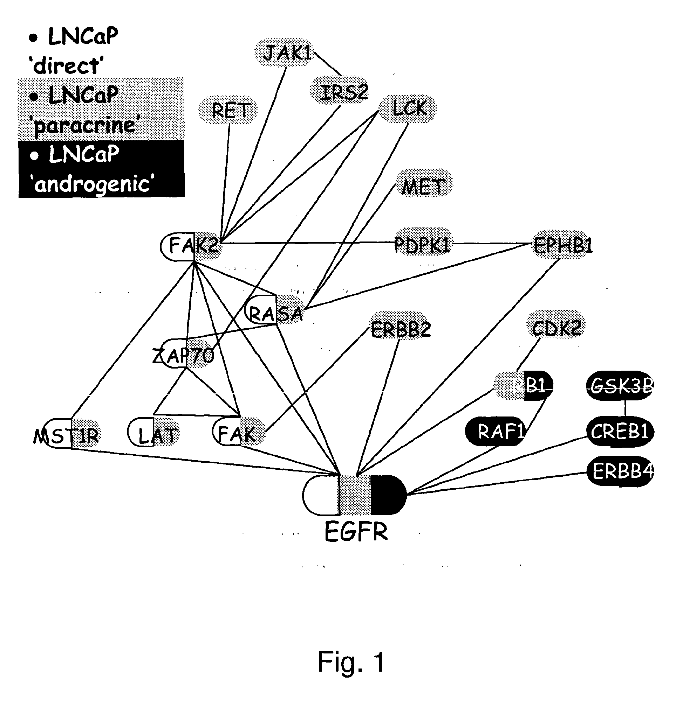

The network connectivity analysis revealed that only one signaling pathway, *i.e*, that mediated by EGFR, was activated by the influence of both osteoblastic cells and androgen treatment (Table 1, figure 1). Hence, these experimental data suggest that targeted inhibition of this particular signaling pathway will simultaneously ablate androgen-driven proliferation of prostate carcinoma cells as well as their survival responses to an osteoblastic microenvironment, thereby providing a biological rationale for first-line use of EGFR inhibition in systemic prevention or treatment of metastatic prostate cancer in the androgen-sensitive stage of the disease.

Moreover, the data suggest the use of EGFR expression, with or without concomitant expression of other tumor cell markers, for detection of circulating tumor cells in bone marrow from prostate cancer patients, also with localized and/or androgen-sensitive disease, as predictive marker for later development of skeletal metastatic disease.

We used two additional experimental setups as biological controls for the LNCaP/OHS cocultures; the first to provide cells that might be more representative of physiological osteoblasts and the second to model regulatory interactions between carcinoma and osteoblastic cells in the androgen-independent stage of prostate cancer.

Non-hematopoietic stem cells in bone marrow are capable of differentiating into a variety of tissue entities, including osteogenic cells of bone tissue [Giordano et al., 2007]. Incubation of mononuclear cells isolated from adult, human bone marrow with mesenchymal stem cell-stimulating medium followed by osteogenic differentiation medium [Colter et al., 2000; Peister et al., 2004] gave rise to cells with osteoblastic characteristics, for example mineral deposition and alkaline phosphatase-secreting activity. These in vitro-differentiated normal osteoblasts were cocultured with LNCaP cells.

Androgen-independent LNCaP-19 cells, which have been derived from LNCaP cells following continuous maintenance in steroid-depleted medium, have been shown to form epithelial-like cell clusters [Gustavsson et al., 2005]. These cells were cocultured with OHS cells.

EGFR-mediated signaling was found increased in LNCaP cells from coculture with osteoblastic cells that had been differentiated from normal, human mesenchymal stem cells, but not in LNCaP-19 cells that were cocultured with OHS cells.

Thus, a first aspect of the invention is Gefinitib for use in the treatment or prevention of metastatic prostate cancer, wherein the treatment is initiated while the primary tumor and/or the metastatic prostate cancer is in the androgen-sensitive stage of the disease.

A second aspect of the invention is use of Gefitinib for the preparation of a medicament for treating or preventing metastatic prostate cancer, wherein the treatment is initiated while the primary tumor and/or metastatic prostate cancer is in the androgen-sensitive stage of the disease.

A third aspect of the invention is a pharmaceutical composition comprising Gefitinib for use in treatment of metastatic prostate cancer, wherein the treatment is initiated while the primary tumor and/or metastatic prostate cancer is in the androgen-sensitive stage of the disease.

### Detailed description

### Brief description of the drawings

Figure 1. Interconnected signaling pathways activated in LNCaP cells by influence of osteoblastic cells or androgen treatment. Two pathway visualization systems were applied to the data set (Table 1), which resulted in almost identical network connectivity maps. The substrate annotations are derived from gene entries in SwissProt. The lines connecting nodes represent interactions of the following types: binding, expression, protein modification, and regulation. Red, yelow, and blue nodes: Substrates activated by the direct, paracrine, and androgenic LNCaP entities, respectively.

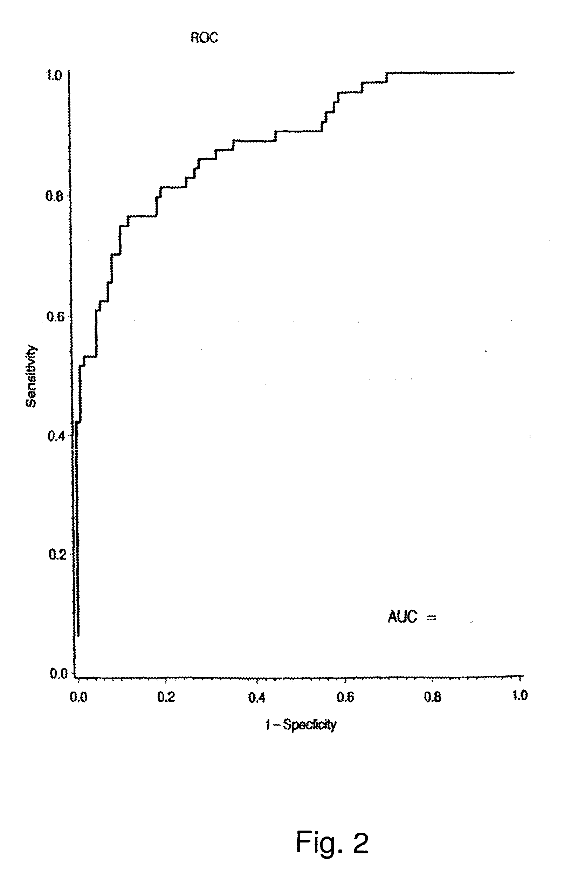

Figure 2. ROC (Receiver Operating Characteristics) curve.

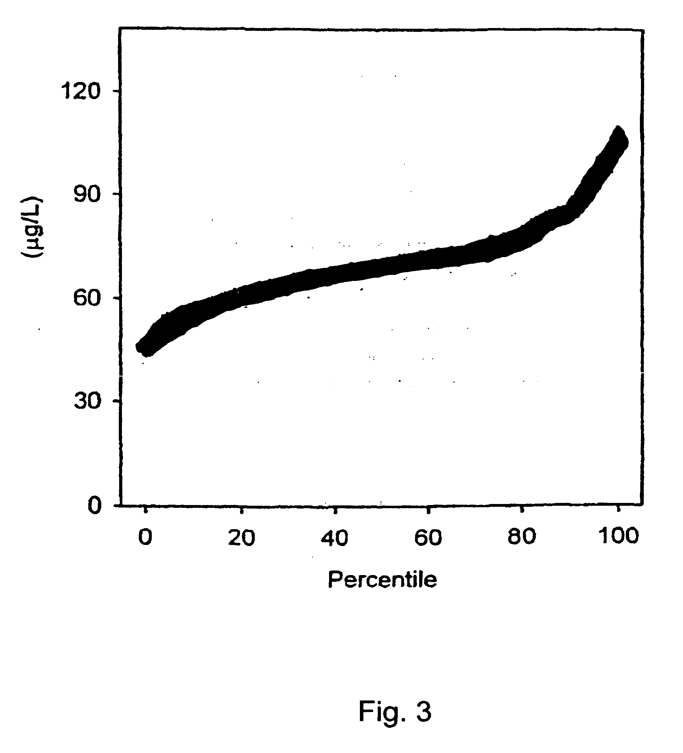

Figure 3. Percentile plot.

### Disclosure of the invention

As described above, the present inventors have studied the regulatory basis underlying establishment of prostate carcinoma cells within an osteoblastic microenvironment using a model system allowing identification of activated intracellular signaling pathways.

The network connectivity analysis of phosphopeptide signatures revealed that only one signaling pathway, i.e, that mediated by EGFR, was activated by the influence of both osteoblastic cells and androgen treatment. These experimental data suggest that targeted inhibition of this particular signaling pathway will simultaneously ablate androgen-driven proliferation of prostate carcinoma cells as well as their survival responses to an osteoblastic microenvironment, thereby providing a biological rationale for first-line use of EGFR inhibition in systemic prevention or treatment of metastatic prostate cancer in the androgen-sensitive stage of the disease.

Moreover, the data also suggest the possible use of EGFR expression, with or without concomitant expression of other tumor cell markers, for detection of circulating tumor cells in bone marrow from prostate cancer patients, also with localized and/or androgen-sensitive disease, as predictive marker for later development of skeletal metastatic disease

Thus, in a first aspect, the invention provides , Gefitinib for use in the treatment or prevention of metastatic prostate cancer, wherein

the treatment is initiated while the cancer is in the androgen-sensitive stage of the disease.

As prostate cancer very often forms metastases in bone, the invention is particularly preferred for use in treating or preventing skeletal metastatic prostate cancer.

The use in treatment may be use in an adjuvant treatment following removal of the primary tumor, neoadjuvant prior to surgery or definitive radiotherapy, or concomitant with radiotherapy.

In a preferred embodiment, the small molecule that targets EGFR is gefitinib N-(3-chloro-4-fluoro-phenyl)-7-methoxy-6-(3-morpholin-4-ylpropoxy)quinazolin-4-amine (Iressa®), .

Dosis and regimen for preferred EGFR inhibitors or EGFR signaling inhibitors or inhibitors of kinases downstream of EGFR kinases are:
*Small molecular tyrosine kinase inhibitors:*
   Gefitinib (Iressa®) oral 250 (-500) mg*1

In a preferred embodiment, the dosis of Gefitinib is reduced as compared to the above listed dosis. Preferably, the dosis is reduced at least 10%, even more preferred at least 25% and most preferred at least 50%. A reduction of dosis may be particular feasible when Gefitinib is combined with androgen ablation treatment.

### Androgen ablation treatment.

In a preferred embodiment of the first aspect, the use further comprises androgen ablation treatment.

Androgen ablation treatment may be achieved surgical removal of the testicles of the patient.

In another embodiment, the use comprising androgen ablation treatment comprises administrating an antiandrogen (testosterone antagonist or LHRH/GnRH analog).

Preferably, the antiandrogen is selected from the group consisting of flutamide (Eulexin), bicalutamide (Casodex) and nilutamide (Nilandron), leuprolin (enanton depot@), buserelin (Suprefact depot) and triptorelin (pamorelin®).

Dosis and regimen for preferred antiandrogens are:

### Testosteron antagonists:

Bicalutamide (Casodex®) oral 50 mg*1
Flutamide (Eulexin®) oral 250mg*3
Flutamide (Flutamid®) oral 250 mg*3

### LHRH/GnRH analoges:

Goserelin (Zoladex®) inj 10,8 mg every 12th week
Leuprorelin (Enanton depot®) inj 11,25 mg every 12th week
Leuprorelin (Procren Depot®) inj 11,25 mg every 12th week
Leuprorelin (Eligard®) inj 22,5 mg every 12th week
Buserelin (Suprefact Depot) inj 6,3 mg every 8th week
Triptorelin (Pamorelin®) inj 11,25 mg every 12th week

In a preferred embodiment, the doses of the preferred antiandrogens are reduced as compared to the above listed doses. Preferably, the dosis is reduced at least 10%, even more preferred at least 25% and most preferred at least 50%.

A second aspect of the invention is the use of Gefitinib for the preparation of a medicament for treating or preventing metastatic prostate cancer, wherein the treatment is initiated while the primary tumor and/or metastatic prostate cancer is in the androgen sensitive stage of the disease. A third aspect of the invention is a pharmaceutical composition comprising Gefitinib for use in the treatment of metastatic prostate cancer. The embodiments of the first aspect also apply to the third aspect of the invention, i.e. also, for use in treatment together with an antiandrogen.

Moreover, the pharmaceutical composition for us according to the invention may comprise both Gefitinib and the antiandrogen.

A second aspect of the invention is the use of Gefitinib for the preparation of a medicament for treating or preventing metastatic prostate cancer. The embodiments of the first aspect also apply to the second aspect of the invention.

A third aspect of the invention is a pharmaceutical composition comprising Gefitinib for use in the treatment of metastatic prostate cancer. The embodiments of the first aspect also apply to the third aspect of the invention, i.e. apply also to use in treatment together with an antiandrogen.

Moreover, the pharmaceutical composition for use according to the invention may comprise both Gefitinib and the antiandrogen.

Throughout the present specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

All patent and non-patent references cited in the present application, are hereby incorporated by reference in their entirety.

The invention will now be described in further details in the following nonlimiting examples.

### Examples

Example 1: EGFR signaling is activated by the influence of both osteoblastic cells and androgen treatment.

### Methods

Cell culture conditions. The LNCaP and OHS cell lines were routinely held in RPMI 1640 medium supplemented with 10% fetal bovine serum (FBS) and 2.0 mM glutamine, defined as growth medium. Different ratios of LNCaP cells to OHS cells had been tested in a series of cocultures to find the optimal culturing conditions [Bratland et al., 2003]. Seventy-two hours before start of experimental incubations, LNCaP and OHS cells were seeded in a 10:1 ratio in RPMI containing 2% charcoal-treated FBS and glutamine. After 48 h, this medium was changed to RPMI containing 0.5% charcoal-treated FBS and glutamine, defined as experimental medium, for another 24 h before experimental incubations were started (at time 0). Monocultures of LNCaP cells were identically incubated prior to experimental incubations. Monocultures of LNCaP, as well as LNCaP/OHS cocultures, were seeded in a total number of 1.0 x 106 cells in 75 cm2 cell flasks and invariably held in 10 ml medium throughout different incubations. At time 0 (start of the experimental incubations), monocultured LNCaP cells were refed with experimental medium supplemented with 100 nM R1881 (methyltrienolone; Life Science Products), a synthetic androgen analog, or with medium conditioned by OHS cells. The conditioned medium had been collected from OHS monocultures that had been seeded in a 10-fold higher number per volume medium compared to the corresponding cell number of the LNCaP/OHS cocultures, and grown for 48 h (relative to time 0) in experimental medium. This medium was subsequently diluted 1:10 in either fresh experimental medium or in medium obtained from standard LNCaP monocultures after 48 h of incubation (relative to time 0) in experimental medium, before the application onto monocultured LNCaP cells.

### Experimental approach

Skeletal metastases from prostate cancer are essentially osteoblastic, as apparent both radiographically and histopathologically, and as consistent with elevated serum level of bone-specific alkaline phosphatase, a marker of osteoblast proliferation, in patients with metastatic prostate cancer [Logothetis & Lin, 2005]. These observations implicate that the biological interaction between the prostate carcinoma cells and osteoblasts contributes to the metastatic progression of prostate cancer. To study the regulatory basis for this heterotypic cellular interaction, a model system allowing identification of activated intracellular signaling pathways was established. We used the human, androgen-sensitive LNCaP prostate carcinoma cell line [Horoszewicz et al., 1983] in coculture with the human, osteoblast-derived OHS cell line [Fodstad et al., 1986], as previously described [Bratland et al., 2003], to experimentally address how osteoblastic cells may influence prostate carcinoma cell biology upon formation of bone metastasis.

To be able to analyze LNCaP signaling pathways activated by direct contact with OHS cells, LNCaP cells were isolated from cocultures by immunomagnetic selection [Forus et al., 1999, Fodstad et al., 2001, Bruland et al., 2005, Tveito et al., 2007]. This rapid and simple procedure enables specific selection of target cells for further analytical applications. Subsequent multiplex profiling of kinase activity was performed using flow-through, porous microarrays with peptide substrates (PamChip® peptide arrays; PamGene International B.V., www.pamgene.com), a novel platform that allows rapid, real-time measurements of phosphopeptide signatures generated by the biological samples.

Insight into regulatory mechanisms underlying establishment of prostate carcinoma cells within an osteoblastic microenvironment might eventually lead to more effective therapies for metastatic disease. Hence, from a therapeutic perspective, we compared the intracellular LNCaP signaling pathways activated by OHS influence with those induced after androgen treatment of LNCaP cells, to suggest whether androgen ablation therapy, as used empirically today, represents a biologically based strategy in prevention and systemic treatment of bone metastasis in prostate cancer.

### THE BIOLOGICAL MODEL

From a clinical point of view, prostate cancer metastasis to bone is a lengthy and complex disease process, which makes it difficult to find adequate laboratory models to recreate all steps involved [Singh & Figg, 2005]. However, we have now developed an experimental model and analytical technology providing relevant information about functional signaling pathways and networks that might initiate this biological process, also within a therapeutic perspective.

In this study, we used human, androgen-sensitive prostate carcinoma cells (LNCaP), considered to represent a validated model for prostate cancer in androgen-sensitive stage, in coculture with human osteoblast-derived cells (OHS). The OHS cell line was originally established from a patient with aggressive osteosarcoma [Fodstad et al., 1986], and it might therefore be argued that it is not fully representative for physiological osteoblasts. However, formation of osteosclerotic (i.e., osteoblastic) lesions following intratibial OHS cell inoculation has previously been demonstrated by radiographic, scintigraphic, and morphologic assessments [Kjonniksen et al., 1994].

### Results

Culturing LNCaP cells with OHS cells, which were seeded in a 10:1 ratio to generate stable cocultures [Bratland 2003], caused substantial change in LNCaP morphology. The spindle-shaped feature of monocultured LNCaP cells was rapidly lost upon direct contact with the OHS cells. In coculture, both cell types appeared rounded, although cytoplasmic processes were still apparent on LNCaP cells. Cellular morphology of the OHS cells, however, seemed to be independent of the culturing conditions.

We have previously evaluated the immunomagnetic cell segregation method for selective isolation of target cells and demonstrated that target cell populations are highly enriched [Forus et al., 1999, Fodstad et al., 2001, Bruland et al., 2005, Tveito et al., 2007]. Although cell fractions binding the MOC-31 (anti-EPCAM) antibody were essentially absent of cells defined as contaminants (i.e., with <5 immunobeads bound to their surface), the analytical application (kinase activity profiling) might be sensitive to their possible presence.

Hence, we screened for phosphopeptide signatures generated by immunoselected as well as monocultured OHS cells. Notably, the resulting substrate phosphorylation patterns were rather similar for the two conditions, but clearly distinguishable from those generated by the LNCaP entities, which argues against significant contamination of OHS cells in MOC-31-positive cell preparations.

Based on numerous observations that prostate carcinoma cells are stimulated by osteoblast-derived factors [Logothetis & Lin, 2005], monocultured LNCaP cells were treated with OHS-conditioned medium to experimentally obtain the biological context of paracrine influence. The profiles of phosphorylated peptides acquired from LNCaP cells were essentially identical whether the OHS-conditioned medium was mixed with fresh medium or with medium conditioned by LNCaP monocultures, although the generated phosphorylation levels were slightly higher for all peptides, with one exception (phospho-RB1), on microarrays incubated with the cells treated with medium conditioned by both cell types. Interestingly, LNCaP morphology appeared unchanged under the influence of OHS-conditioned media.

Androgens are critical regulators of prostate carcinoma progression. Most patients respond to androgen ablation therapy, but only temporarily, also at bone metastasis sites. Until recently, the regulatory program mediated by the androgen receptor in prostate cancer has been elusive [Dehm & Tindall, 2006]. To simulate the complex processes involved in aberrant activation of the androgen signaling axis upon disease progression of prostate cancer [Feldman & Feldman, 2001, Attard et al., 2006], our experimental setting included LNCaP cells treated with a synthetic androgen analog (R1881, 100 nM) to observe whether androgen receptor-mediated signaling pathways might be different from those activated by OHS-directed influence.

The LNCaP samples were given denotations in accordance with their biological context: 'direct' (cells from LNCaP/OHS cocultures), 'paracrine 1' (cells treated with OHS-conditioned medium mixed with fresh medium), 'paracrine 2' (cells treated with OHS-conditioned medium mixed with medium conditioned by LNCaP cells), and 'androgenic' (cells treated with the synthetic androgen analog), in addition to baseline (untreated reference cells).

### KINASE ACTIVITY PROFILING

To identify regulatory mechanisms underlying the affinity of prostate cancer to bone, the substrate phosphorylation state generated by each LNCaP entity ('direct', 'paracrine', 'androgenic') was calculated with respect to baseline. Based on the assumption that biologically relevant signaling events implicated in the metastatic phenotype require sustained activation, 48 h incubation times were used for all experimental LNCaP contexts. The identified substrates showed increases in phosphorylation level within a broad range (Table 1).

**Table 1: List of peptides with increased phosphorylation levels generated by the various LNCaP entities. For each substrate, position of phosphorylation sites within the protein is indicated. '-' denotes that the change in peptide phosphorylation level was not found to be significant. Fold changes relative to LNCaP baseline sample are listed**

| Phosphopeptide | 'direct' | | 'paracrine' | | 'androgenic' | |
|---|---|---|---|---|---|---|
| | | | fold change (log2) | | | |
| EGFR_y1197 | 2.16 | | 1.84 | | 1.94 | |
| EGFR_y1110 | 1.86 | | 2.34 | | - | |
| MST1R_y1353 | | 2.20 | | 2.86 | | - |
| MST1R_y1356/y1360 | | 2.01 | | 3.38 | | - |
| FAK_y576/y577 | 1.03 | | 1.39 | | - | |
| FAK2_y579/y580 | 0.85 | | 1.21 | | - | |
| LAT_y200 | 2.94 | | 3.49 | | - | |
| ZAP70_y492/y493 | 1.21 | | 1.93 | | - | |
| RASA_y460 | 0.96 | | 1.63 | | - | |
| RET_y1029 | - | | 1.76 | | - | |
| IRS2_y919 | - | | 5.56 | | - | |
| JAK1_y1022/y1023 | - | | 2.05 | | - | |
| LCK_y394 | - | | 1.91 | | - | |
| MET_y1230/y1234/y1235 | - | | 1.57 | | - | |
| PDPK1_y9 | - | | 1.82 | | - | |
| PDPK1_y373/y376 | - | | 1.82 | | - | |
| EPHB1_y778 | - | | 2.11 | | - | |
| ERBB2_y877 | - | | 1.67 | | - | |
| ERBB2_y1248 | | - | | 1.56 | | - |
| CDK2_t14/y15 | - | | 1.86 | | - | |
| RB1_s807/s811 | - | | 3.39 | | 3.07 | |
| RAF1_s337/s338/y339/y340 | - | | - | | 1.14 | |
| GSK3B_y216 | - | | - | | 1.15 | |
| CREB1_y134/s133 | - | | - | | 1.47 | |
| ERBB4_y1284 | | - | | - | | 3.31 |
| CHRNB1_y390 | | 0.95 | | 1.57 | | 1.57 |
| LTK_y772/y776/y777 | 0.96 | | - | | 1.20 | |
| DDR1_y792/y796/y797 | 0.91 | | - | | - | |
| MAPK10 | 0.98 | | - | | - | |
| MAPK12_t183/y185 | 2.23 | | - | | - | |
| PECAM1_y713 | | 0.87 | | 2.06 | | - |
| PRRX2_y214 | 0.93 | | 1.48 | | - | |
| ANXA1_y20/t23 | - | | 2.41 | | - | |
| CD79A_y182/y188 | - | | 1.75 | | - | |
| CTTN1_y477/y483 | - | | 2.01 | | - | |
| CTTN1_y499 | - | | 2.13 | | - | |
| ENO2_y43 | - | | 1.85 | | - | |
| EPHA2_y772 | - | | 1.99 | | - | |
| EPHA7_y608/y614 | - | | 2.27 | | - | |
| EPOR_y368 | - | | 2.08 | | - | |
| EPOR_y426 | - | | 2.29 | | - | |
| FER_y714 | - | | 1.66 | | - | |
| FES_y713 | - | | 2.20 | | - | |
| FGFR2_y769 | - | | 1.77 | | - | |
| FGFR3_y760 | - | | 2.23 | | - | |
| FRK_y387 | - | | 2.02 | | - | |
| LAT_y255 | - | | 1.54 | | - | |
| NTRK2_y702/y706/y707 | - | | 1.43 | | - | |
| PDGFRB_y579/y581 | - | | 3.87 | | - | |
| PDGFRB_y716 | | - | | 1.78 | | - |
| PIK3R1_y607/s608 | - | | 2.09 | | - | |
| PXN_y31 | - | | 1.88 | | - | |
| PXN_y118 | - | | 2.07 | | - | |
| TEC_y519 | - | | 1.68 | | - | |
| PFKFB1_s33 | - | | - | | 1.36 | |
| PTPN11_y542 | | - | | - | | 2.94 |
| SYN1_s9 | - | | - | | 1.20 | |

Each individual phosphopeptide signature was considered to represent a subset of the information flow through the globally activated signaling network of the particular LNCaP entity. To dissect how this information was directed, and in accordance with current recommendations [Petricoin III et al., 2005], we applied bioinformatics analysis methodologies routinely used for analysis of gene expression microarrays. By using such an approach, we assumed that phosphorylation events that appeared simultaneously might be interlinked and provide information about pathway connectivity [Sevecka & MacBeath, 2006].

By applying these assumptions, the network interaction analysis omitted phosphorylated substrates that did not appear within any signaling pathway when defined by the interaction types delineated in Methods. Comparison of Table 1 with figure 1 indicates which phosphopeptides were left out. As illustrated by figure 1, the resulting network connectivity map showed that signaling pathways involved in cell adhesion and motility were activated in the 'direct' LNCaP entity, whereas the 'paracrine' entity additionally phosphorylated substrates involved in cell proliferation. Activation of similar but also completely unrelated proliferation pathways was observed with the 'androgenic' entity. Interestingly, only one signaling pathway, i.e, mediated by EGFR, was activated by the influence of both osteoblastic cells and androgen treatment.

### Conclusion

The network connectivity analysis revealed that only one signaling pathway, i.e, mediated by EGFR, was activated by the influence of both osteoblastic cells and androgen treatment. Based on these experimental data, we therefore hypothesize that targeted inhibition of this particular signaling pathway may simultaneously ablate androgen-driven proliferation of prostate carcinoma cells as well as their survival responses to an osteoblastic microenvironment, thereby providing a biological rationale for first-line use of EGFR inhibition in systemic prevention or treatment of metastatic prostate cancer in the androgen-sensitive stage of the disease.

Moreover, our data also suggest the possible use of EGFR expression, with or without concomitant expression of other tumor cell markers, for detection of circulating tumor cells in bone marrow from prostate cancer patients, also with localized and/or androgen-sensitive disease, as predictive marker for later development of skeletal metastatic disease.

### Example 2: EGFR signaling is activated in androgen-sensitive prostate carcinoma cells by the influence of normal, in vitro-differentiated osteoblasts

### Introduction

Non-hematopoietic stem cells in bone marrow are capable of differentiating into a variety of tissue entities, including osteogenic cells of bone tissue [Giordano et al., 2007]. Incubation of mononuclear cells isolated from adult, human bone marrow with mesenchymal stem cell-stimulating medium followed by osteogenic differentiation medium [Colter et al., 2000; Peister et al., 2004] gave rise to cells with osteoblastic characteristics, for example mineral deposition and alkaline phosphatase-secreting activity. These in vitro-differentiated normal osteoblasts were cocultured with LNCaP cells.

### Results

We applied the immunomagnetic cell separation method for selective isolation of the LNCaP cells from the cocultured osteoblastic cells and subjected the isolated carcinoma cells to analysis by conventional western immunoblotting. Increased expression levels of EGFR phosphorylated on tyrosine 1173 were found in the isolated LNCaP cells.

### Conclusion

Activation of EGFR signaling was again found induced in prostate carcinoma cells under influence of osteoblastic cells, this time osteoblasts that had been differentiated from normal, human mesenchymal stem cells.

### Example 3: Inability of osteoblastic cells to activate EGFR signaling in androgen-independent prostate carcinoma cells

### Introduction

Androgen-independent LNCaP-19 cells had been derived from LNCaP cells following continuous maintenance in steroid-depleted medium [Gustavsson et al., 2005]. These cells were cocultured with OHS cells.

### Results

We applied the immunomagnetic cell separation method for selective isolation of the LNCaP-19 cells from the cocultured OHS cells and subjected the isolated carcinoma cells to analysis by conventional western immunoblotting. In clear contrast to the situation in androgen-sensitive prostate carcinoma cells (the maternal LNCaP cells), phosphorylation of EGFR on tyrosine 1173 was completely absent in LNCaP-19 cells that had been cocultured with osteoblastic cells.

### Conclusion

Given that EGFR is phosphorylated in the androgen-sensitive carcinoma cells upon influence of osteoblasts but not in the androgen-independent derivative cells, a functional androgen signaling axis [Scher & Sawyers, 2005; Attard et al., 2006] is probably permissive for activity of this particular pathway in prostate cancer.

Separately, EGFR may also facilitate androgen receptor-driven activity in prostate cancer at the level of target gene transcription [Gregory et al., 2004]. Androgen receptor pathway genes, identified by system-level analysis of gene expression in primary tumor specimens from therapy-naive prostate cancer patients, were reported to be down-regulated in lymph node metastases from the patients [Hendriksen et al., 2006]. This finding further supports the assumption that the regulatory control by the androgen receptor on carcinoma cell biology is lost in the process of prostate cancer metastasis.

### Perspectives

Of importance, our experimental data suggests that targeted inhibition of signaling pathways directed by EGFR may simultaneously ablate androgen-driven proliferation of prostate carcinoma cells and the survival responses within an osteoblastic microenvironment. It equally provides a biological rationale for the use of EGFR inhibition in systemic prevention or treatment of metastatic prostate cancer in the androgen-sensitive stage of the disease. Intriguingly, the therapeutic concept of EGFR inhibition in hormone-refractory prostate cancer has recently been evaluated; however, in initial studies addressing the use of single-agent therapies in patients with androgen- resistant disease, neither a receptor-blocking antibody nor a small-molecular tyrosine kinase inhibitor showed clinically significant activity [de Bono et al., 2007; Agus et al., 2007; Canil et al., 2005]. Extrapolating from our data, we believe the loss of functional signaling governed by EGFR in androgen-independent prostate carcinoma cells may provide a biological explanation for the poor treatment efficacy in these trials. However, if exploitable in patients with therapy-naïve prostate cancer, inhibitory EGFR targeting might be incorporated into treatment schedules with a potential reduction of the alternative requirement of long-term androgen depletion, a reduction in relared side effects, and, intriguingly, the potential for an improvement in patient survival.

### Example 4: Detection, prediction and monitoring

### Introduction

Clinical and experimental evidence suggests that epithelial tumor cells are able to disseminate to secondary organs at an early stage of primary tumor development. The red bone marrow represents an important indicator organ of hematogenous micrometastatic spread of carcinomas. According to current concept, however, disseminated tumor cells detected in the bone marrow are not able to grow as distant metastatic lesions unless they possess certain biological characteristics that may mediate proliferative responses upon colonization of the secondary organ.

The classical, experimental works on mechanisms of tumor metastasis demonstrated that only a small subset of cells within the parental population is capable of metastasizing and that the cellular composition of secondary tumors differs from that of the primaries. Cellular properties that are crucial for initiation of distant tumor growths, however, may be obscured by clonal heterogeneity of the fully established metastatic lesion. These findings are also in accordance with the contemporary concept of cancer stem cells. Hence, it is biologically relevant to analyze molecular properties that determine the affinity of epithelial tumor cells to the bone marrow and identify biomarkers that may correlate with the ability of distant growth and/or therapies directed against occult or established metastatic disease.

### Immunomagnetic target cell segregation from bone marrow

MOC-31 (IQ Corporation BV, Groningen, the Netherlands) is an IgG1 class antibody that binds to the EPCAM antigen, which is consistently expressed in most epithelial cells [de Jonge et al., 1993]. The antibody is conjugated to superparamagnetic monodisperse particles coated with polyclonal sheep-antimouse IgG particles (Dynabeads SAM-450; Dynal A.S., Oslo, Norway), as recommended by the manufacturer.

Samples of red bone marrow (~15 ml) are acquired by aspiration from the upper iliac crest of prostate cancer patients. After Lymphoprep (Nycomed, Oslo, Norway) density gradient centrifugation (1000 g for 10 min), mononuclear cells from the interface layer are collected, washed, and resuspended in 1% human serum albumin in 0.9% NaCl (HSA/PBS), then counted and diluted to a final concentration of ~10⁷ cells/ml for immunomagnetic separation.

All solutions and cell preparations are kept on ice during the whole procedure to avoid nonspecific binding of immunobeads. First, MOC-31-coated beads are added at a ratio of 10:1 to total number of suspended cells, and the suspensions are incubated for 30 min at 4°C on a rotating mixer. The cells are subsequently diluted in HSA/PBS to a final volume of 3.0 ml and left in a magnet holder for 2 min, and the supernatants, containing unbound cells, are decanted. The remaining cell-bead rosettes, trapped on the wall of the test tubes by the magnet, are washed three times with surplus volume of HSA/PBS to remove any contaminating material. Of the remaining positive cell fractions of ~200 µl, 20 µl aliquots are examined by light microscopy for the principal presence of cells with ≥5 immunobeads bound to their surface (i.e., cell-bead rosettes). Quality control of positive and negative cell fractions has been documented previously [Forus et al., 1999; Fodstad et al., 2001, Bruland et al., 2005, Tveito et al., 2007].

### Characterization of tumor cells isolated from bone marrow

Fluorescent latex microparticles (Molecular Probes Europe, Leiden, the Netherlands) are conjugated with different antibodies (against EGFR, ERBB2, ERBB4, MST1R (RON), FAK, MET (HGFR), RET, RAF and CREB1) and used in a double staining procedure to show that cells magnetically selected with SAM-450 beads coated with MOC-31 also bind latex particles with antibodies targeting one of the other epitopes/antigens, thus providing additional evidence that the rosetted cells are indeed tumor cells and that their presence in bone marrow might predict later development of bone metastatic disease.

### Immunocytochemistry for detection of circulating tumor cells in bone marrow

Immunocytochemistry is done on cytospins from mononuclear cells suspensions of bone marrow aspirates using the APAAP technique (DAKO, Copenhagen, Denmark). The detected cells are double-stained with different antibodies (against EGFR, ERBB2, ERBB4, MST1R (RON), FAK, MET (HGFR), RET, RAF and CREB1) to detect tumor cells that might predict later development of bone metastatic disease.

### Immunohistochemistry of biopsy or surgical samples

Although a small subset only of cells within the primary tumor is capable of metastasizing, in accordance with the contemporary concept of cancer stem cells, it might be therapeutically relevant to identify cells within the primary tumor that are likely to metastasize. This might be accomplished by means of immunohistochemistry of biopsy or surgical specimens with different antibodies (against EGFR, ERBB2, ERBB4, MST1R (RON), FAK, MET (HGFR), RET, RAF and CREB1).

### Kinase activity profiling of tumor samples

To identify the regulatory basis for the metastasizing capacity of prostate carcinoma cells, this cell population may be analyzed for activated intracellular signaling pathways. This might be technically challenging, however, with a limited number of target cells available for the purpose. One possible analytical approach is described.

Tumor cells (isolated from biopsy or surgical material from the primary tumor or from bone marrow) are lysed in M-PER Mammalian Extraction Reagent containing Halt Phosphatase Inhibitor Cocktail and EDTA-free Halt Protease Inhibitor Cocktail (Pierce Biotechnology, Inc.). Reference lysates (baseline samples) are made from monocultured LNCaP cells or biopsy or surgical material of normal prostate tissue.

The peptide substrate array technology allows functional comparison of biological samples without prior knowledge of the activity pathways influenced by the experimental manipulations. The high-throughput format of the PamChip® technology (PamGene International B.V., www.pamgene.com) is based on the use of a porous, three-dimensional aluminum-oxide material as solid support for the substrates. The samples lysates are actively pumped through the interconnected capillary pores of the arrays to allow contact with the reactive surface, which is increased ~500-fold compared to two-dimensional geometry arrays, for enzymatic reaction with the peptide substrates. The phosphorylation kinetics is therefore rapid and can be completed within few minutes, allowing the generation of spot images to be followed in real-time. Each array contains ~140 peptides spotted in duplicate, and these peptides consist of 13, 14 or 15 amino acids with sites for phosphorylation, mainly tyrosine.

The image information is converted using BioNavigator software (PamGene International B.V.). For each spot on the array, signal intensity after background subtraction is calculated and used for further analysis. Data normalization of mean signal intensity from duplicate spots and subsequent comparison analyses are conducted using GeneSpring software (Agilent Technologies, www.home.agilent.com). All values are normalized to the calculated mean value of all substrate phosphorylation intensities in the baseline sample.

Several different pathway visualization systems can be used to create information about pathway connectivity (*e.g*., PathwayArchitect software (Stratagene Corp., www.stratagene.com; Strand Life Sciences Pvt. Ltd., www.avadis.strandgenomics.com), PathwayStudio software (Ariadne Genomics, www.ariadnegenomics.com). The peptide identifications can be visualized through a direct interaction network, defined to show all interactions between peptides that are of the following types: binding, expression, protein modification, and regulation. Pathways may also be created directly from the ResNet database (Ariadne Genomics) and ranked by the hypergeometric probability factor where all interactions are selected on the criterion of the highest number of proteins being involved in a linear pathway or sub-pathway.

### Systemic therapy

Micrometastasis status has been proposed as an entry in the TNM classification system of the International Union Against Cancer (UICC) as a prognostic factor for several types of solid cancers. In patients with primary breast cancer the presence of bone marrow micrometastases is significantly associated with shorter survival, but is not an independent prognostic factor, as shown by short-term as well as long-term follow-up studies.

A recent publication on patients with localized prostate cancer treated with definitive radiotherapy (*i.e*., curatively intended therapy) showed that the presence of circulating tumor cells in bone marrow at time of diagnosis was associated with increased risk of developing distant metastases [Berg *et al.,* 2007]. Hence, given that the right patient population is identified, adjuvant treatment after curatively intended therapy (surgery, radiotherapy, other therapeutic modalities) may be shown beneficial. This must be proven in prospective, randomized trails.

Moreover, since our experimental data show that the signalling pathway mediated by EGFR was activated by the influence of both osteoblastic cells and androgen treatment, we hypothesize that targeted inhibition of EGFR signalling may ablate prostate carcinoma cells' survival responses to an osteoblastic microenvironment as well as androgens. Hence, in a metastatic setting, palliative, systemic treatment with EGFR inhibitor or EGFR signaling inhibitor or inhibitor of kinases downstream of EGFR kinases, in the absence or presence of androgen ablation, might be beneficial, also in first-line setting (when the tumor is still androgen-sensitive).

### REFERENCES

Agus DB, Sweeney CJ, Morris MJ et al. Efficacy and safety of single-agent pertuzumab (rhuMAb 2C4), a human epidermal growth factor dimerization inhibitor, in castration-resistant prostate cancer after progression from taxane-based therapy. J Clin Oncol 2007; 25, 675-81.
Attard G, Sarker D, Reid A, Molife R, Parker C, de Bono JS. Improving the outcome of patients with castration-resistant prostate cancer through rational drug development. Br J Cancer 2006; 95, 767-74.
Berg A, Berner A, Lilleby W, Bruland OS, Fossa SD, Nesland JM, Kvalheim G. Impact of disseminated tumor cells in bone marrow at diagnosis in patients with nonmetastatic prostate cancer treated by definitive radiotherapy. Int J Cancer 2007; 120, 1603-9.
Bratland Å, Ragnhildstveit E, Bjørnland K, Andersen K, Maelandsmo GM, Fodstad 0, Saatcioglu F, Ree AH. The metalloproteinase inhibitor TIMP-2 is down-regulated by androgens in LNCaP prostate carcinoma cells. Clin Exp Metastasis 2003;20:541-7.
Bruland OS, Hoifodt H, Saeter G, Smeland S, Fodstad O. Hematogenous micrometastases in osteosarcoma patients. Clin Cancer Res 2005;11:4666-73.
Canil CM, Moore MJ, Winquist E et al. Randomized phase II study of two doses of gefitinib in hormone-refractory prostate cancer: a trial of the National Cancer Institute of Canada-Clinical Trials Group. J Clin Oncol 2007; 23, 455-60.
Colter DC, Class R, DiGirolamo CM, Prockop DJ. Rapid expansion of recycling stem cells in cultures of plastic-adherent cells from human bone marrow. Proc Natl Acad Sci USA 2000; 97, 3213-8.
de Bono JS, Bellmunt J, Attard G et al. Open-label, phase II study evaluating the efficacy and safety of two doses of pertuzumab in castrate chemotherapy-naïve patients with hormone-refractory prostate cancer. J Clin Oncol 2007; 25, 257-62.
Dehm SM, Tindall DJ. Molecular regulation of androgen action in prostate cancer. Cell Biochem 2006;Mar 3 [Epub ahead of print].
de Jonge MW, Kosterink JG, Bin YY, Bulte JW, Kengen RA, Piers DA, The TH, de Leij L. Radioimmunodetection of human small cells lung carcinoma xenografts in the nude rat using 111in-labelled monoclonal antibody MOC-31. Eur J Cancer 1993;29A: 1885-90.
Feldman BJ, Feldman D. The development of androgen-independent prostate cancer. Nat Rev Cancer 2001; 1, 34-45.
Fodstad Ø, Brøgger A, Bruland 0, Solheim OP, Nesland JM, Pihl A. Characteristics of a cell line established from a patient with multiple osteosarcoma, appearing 13 years after treatment for bilateral retinoblastoma. Int J Cancer 1986; 38: 33-40.
Fodstad 0, Faye R, Høifødt HK, Skovlund E, Aamdal S. Immunobead-based detection and characterization of circulating tumor cells in melanoma patients. Recent Results Cancer Res 2001;158:40-50.
Forus A, Høifødt HK, Øverli GET, Myklebost O, Fodstad Ø. Sensitive fluorescent in situ hybridisation method for the characterisation of breast cancer cells in bone marrow aspirates. J Clin Pathol Mol Pathol 1999;52:68-74.
Giordano A, Galderisi U, Marino IR. From the laboratory bench to the patient's bedside: an update on clinical trials with mesenchymal stem cells. J Cell Physiol 2007; 211, 27-35.
Gregory CW, Fei X, Ponguta LA et al. Epidermal growth factor increases coactivation of the androgen receptor in recurrent prostate cancer. J Biol Chem 2004; 279, 7119-30.
Gustavsson H, Welén K, Damber JE. Transition of an androgen-dependent human prostate cancer cell line into an androgen-independent subline as associated with increased angiogenesis. Prostate 2005; 62, 364-73.
Gregory CW, Fei X, Ponguta LA et al. Epidermal growth factor increases coactivation of the androgen receptor in recurrent prostate cancer. J Biol Chem 2004; 279, 7119-30.
Hendriksen PJ, Dits NF, Kokame K et al. Evolution of the androgen receptor pathway during progression of prostate cancer. Cancer Res 2006; 66, 5012-20.
Horoszewicz JS, Leong SS, Kawinski E, Karr JP, Rosenthal H, Chu TM, Mirand EA, Murphy GP. LNCaP model of human prostatic carcinoma. Cancer Res 1983;43:1809-18.
Kjønniksen I, Winderen M, Bruland Ø, Fodstad 0. Validity and usefulness of human tumor models established by intratibial cell inoculation in nude rats. Cancer Res 1994; 54, 1715-9.
Logothetis CJ, Un SH. Osteoblasts in prostate cancer metastasis to bone. Nat Rev Cancer 2005; 5:21-8.
Peister A, Melland JA, Wang M, Tucker HA, Prockop DJ. Stable transfection of MSCs by electroporation. Gene Therapy 2004; 11, 224-8.
Petricoin III EF, Bichsel VE, Calvert VS, Espina V, Winters M, Young L, Belluco C, Trock BJ, Lippman M, Fishman DA, Sgroi DC, Munson PJ, Esserman U, Liotta LA. Mapping molecular networks using proteomics: a vision for patient-tailored combination therapy. J Clin Oncol 2005; 23, 3614-21.
Scher HI, Sawyers CL. Biology of progressive, castration-resistant prostate cancer: directed therapies targeting the androgen-receptor signaling axis. J Clin Oncol 2005; 32, 8253-61.
Sevecka M, MacBeath G. State-based discovery: a multidimensional screen for small-molecule modulators of EGF signaling. Nat Methods 2006; 3, 825-31.
Singh AS, Figg WD. In vivo models of prostate cancer metastasis to bone. J Urol 2005; 174, 820-6.
Tveito S, Maelandsmo GM, Hoifodt HK, Rasmussen H, Fodstad O. Specific isolation of disseminated cancer cells - a new method permitting sensitive detection of target molecules of diagnostic and therapeutic value. Clin Exp Metastasis 2007; in press.

## Claims

1. Gefitinib for use in the treatment or prevention of metastatic prostate cancer wherein said treatment is initiated while the primary tumor and/or the metastatic prostate cancer is in the androgen-sensitive stage of the disease.

2. Gefitinib for use according to claim 1, wherein the treatment is an adjuvant treatment following removal (surgery, radiotherapy, other therapy modalities) of the primary tumor.

3. Gefitinib for use according to any of the preceding claims, wherein said treatment further comprises androgen ablation.

4. Gefitinib for use according to claim 3, wherein said androgen ablation comprises administrating an antiandrogen selected from the group consisting of flutamide (Eulexin), bicalutamide (Casodex) and nilutamide (Nilandron), leuprolin (Enanton depot@), buserelin (Suprefact depot) and triptorelin (Pamorelin®).

5. Use of Gefitinib for the preparation of a medicament for treating or preventing metastatic prostate cancer, wherein the treatment is initiated while the primary tumor and/or the metastatic prostate cancer is in the androgen-sensitive stage of the disease.

6. The use according to claim 5, wherein said treatment further comprises androgen ablation.

7. The use according to claim 6, wherein said androgen ablation comprises administrating an antiandrogen selected from the group consisting of flutamide (Eulexin), bicalutamide (Casodex) and nilutamide (Nilandron), leuprolin (Enanton depot@), buserelin (Suprefact depot) and triptorelin (Pamorelin®).

8. A pharmaceutical composition comprising Gefitinib for use in the treatment of metastatic prostate cancer, wherein the treatment is initiated while the primary tumor and/or the metastatic prostate cancer is in the androgen-sensitive stage of the disease.

9. The pharmaceutical composition for use according to claim 8, wherein said treatment further comprises androgen ablation.

10. The pharmaceutical composition for use according to claim 9, wherein said androgen ablation comprises administrating an antiandrogen selected from the group consisting of flutamide (Eulexin), bicalutamide (Casodex) and nilutamide (Nilandron), leuprolin (Enanton depot@), buserelin (Suprefact depot) and triptorelin (Pamorelin®).

## Patentansprüche

1. Gefitinib für die Verwendung bei der Behandlung oder Verhinderung von metastasiertem Prostatakrebs, wobei die Behandlung eingeleitet wird, während sich der Primärtumor und/oder der metastasierte Prostatakrebs im androgenempfindlichen Stadium der Krankheit befinden/ befindet.

2. Gefitinib für die Verwendung nach Anspruch 1, wobei es sich bei der Behandlung um eine adjuvante Behandlung nach Entfernung (Operation, Bestrahlungstherapie, andere Behandlungsmodalitäten) des Primärtumors handelt.

3. Gefitinib für die Verwendung nach einem der vorherigen Ansprüche, wobei die Behandlung des Weiteren eine Androgenablation umfasst.

4. Gefitinib für die Verwendung nach Anspruch 3, wobei die Androgenablation das Verabreichen eines Antiandrogens umfasst, das aus der Gruppe ausgewählt ist, die aus Flutamid (Eulexin), Bicalutamid (Casodex) und Nilutamid (Nilandron), Leuprolin (Enanton depot@), Buserelin (Suprefact depot) und Triptorelin (Pamorelin®) besteht.

5. Verwendung von Gefitinib für die Zubereitung eines Medikaments zum Behandeln oder Verhindern von metastasiertem Prostatakrebs, wobei die Behandlung eingeleitet wird, während sich der Primärtumor und/oder der metastasierte Prostatakrebs im androgenempfindlichen Stadium der Krankheit befinden/befindet.

6. Verwendung nach Anspruch 5, wobei die Behandlung des Weiteren eine Androgenablation umfasst.

7. Verwendung nach Anspruch 6, wobei die Androgenablation das Verabreichen eines Antiandrogens umfasst, das aus der Gruppe ausgewählt ist, die aus Flutamid (Eulexin), Bicalutamid (Casodex) und Nilutamid (Nilandron), Leuprolin (Enanton depot@), Buserelin (Suprefact depot) und Triptorelin (Pamorelin®) besteht.

8. Pharmazeutische Zusammensetzung, die Gefitinib aufweist, für die Verwendung bei der Behandlung von metastasiertem Prostatakrebs, wobei die Behandlung eingeleitet wird, während sich der Primärtumor und/oder der metastasierte Prostatakrebs im androgenempfindlichen Stadium der Krankheit befinden/befindet.

9. Pharmazeutische Zusammensetzung für die Verwendung nach Anspruch 8, wobei die Behandlung des Weiteren eine Androgenablation umfasst.

10. Pharmazeutische Zusammensetzung für die Verwendung nach Anspruch 9, wobei die Androgenablation das Verabreichen eines Antiandrogens umfasst, das aus der Gruppe ausgewählt ist, die aus Flutamid (Eulexin), Bicalutamid (Casodex) und Nilutamid (Nilandron), Leuprolin (Enanton depot@), Buserelin (Suprefact depot) und Triptorelin (Pamorelin®) besteht.

## Revendications

1. Géfitinib destiné à être utilisé dans le traitement ou la prévention du cancer métastatique de la prostate, ledit traitement étant initié alors que la tumeur primitive et/ou le cancer métastatique de la prostate se trouvent au stade de la maladie sensible aux androgènes.

2. Géfitinib destiné à être utilisé selon la revendication 1, le traitement étant un traitement adjuvant après une extraction de la tumeur primaire (chirurgie, radiothérapie, autres modalités thérapeutiques).

3. Géfitinib destiné à être utilisé selon l'une quelconque des revendications précédentes, ledit traitement comprenant en outre la suppression androgénique.

4. Géfitinib destiné à être utilisé selon la revendication 3, ladite suppression androgénique comprenant l'administration d'un antiandrogène choisi dans le groupe constitué par le flutamide (Eulexine) le bicalutamide (Casodex) et le nilutamide (Nilandron), la leuproline (Enanton depot@), la buséréline (Suprefact depot) et la triptoréline (Pamorelin®).

5. Utilisation de géfitinib pour la préparation d'un médicament destiné au traitement ou à la prévention du cancer métastatique de la prostate, le traitement étant initié alors que la tumeur primitive et/ou le cancer métastatique de la prostate se trouvent au stade de la maladie sensible aux androgènes.

6. Utilisation selon la revendication 5, ledit traitement comprenant en outre la suppression androgénique.

7. Utilisation selon la revendication 6, ladite suppression androgénique comprenant l'administration d'un antiandrogène choisi dans le groupe constitué par le flutamide (Eulexine) le bicalutamide (Casodex) et le nilutamide (Nilandron), la leuproline (Enanton depot@), la buséréline (Suprefact depot) et la triptoréline (Pamorelin®).

8. Composition pharmaceutique comprenant du géfitinib et destinée à être utilisée dans le traitement du cancer métastatique de la prostate, le traitement étant initié alors que la tumeur primitive et/ou le cancer métastatique de la prostate se trouvent au stade de la maladie sensible aux androgènes.

9. Composition pharmaceutique destinée à être utilisée selon la revendication 8, ledit traitement comprenant en outre la suppression androgénique.

10. Composition pharmaceutique destinée à être utilisée selon la revendication 9, ladite suppression androgénique comprenant l'administration d'un antiandrogène choisi dans le groupe constitué par le flutamide (Eulexine) le bicalutamide (Casodex) et le nilutamide (Nilandron), la leuproline (Enanton depot®), la buséréline (Suprefact depot) et la triptoréline (Pamorelin®).
